Europäisches Patentamt

European Patent Office

Office européen des brevets

(19)

(11) Publication number: **0 150 602**
A2

# EUROPEAN PATENT APPLICATION

(12)

(21) Application number: 84308828.7

(22) Date of filing: 17.12.84

(51) Int. Cl.⁴: **C 12 M 1/40,** G 01 N 27/30, **C 07 F 15/00**

(30) Priority: 20.12.83 GB 8333917

(43) Date of publication of application: 07.08.85
Bulletin 85/32

(84) Designated Contracting States: AT BE CH DE FR GB IT LI LU NL SE

(71) Applicant: Genetics International, Inc., 50 Milk Street Fifteenth Floor, Boston, MA 02109 (US)

(72) Inventor: Hill, Hugh Allen Oliver, 9 Clover Close, Oxford OX2 9JH (GB)

(74) Representative: Clifford, Frederick Alan et al, MARKS & CLERK 57/60 Lincoln's Inn Fields, London WC2A 3LS (GB)

(54) Carbon-boron compounds in electron transfer electrodes.

(57) The present specification discloses a mediator compound for use with an electron transfer electrode the said compound containing carbon and boron.

Conveniently the mediator compound is a metallocarborane.

In general, the metallocarboranes have greater thermal stability and resistance to chemical attack that the respective metallocene analogues. More importantly they allow a much wider range of electrochemical potentials to be assessed.

The following metallocarboranes and complex ions are employed:

(a) the bisdicarbollyl complexes of $d^3$, $d^5$, $d^6$, $d^7$, $d^8$ and $d^9$ transition metal ions,

(b) mixed complexes in which a dicarbollyl ligand formally replaces a cyclopentadienyl ligand in a metallocene,

(c) bidentate and polydentate complexes containing the dicarbacanastide ligand; and

(d) dinuclear and polynuclear complexes containing one or more cyclopentadienyl ligands.

As with the previous mediator compounds these compounds are capable of transferring charge from an enzyme or enzyme complex to an electrode surface when the enzyme is catalytically active.

○ B
● C
○ H

EP 0 150 602 A2

M&C FOLIO: 230P47244X                    WANGDOC: 0287s


Title:   CARBON-BORON COMPOUNDS IN ELECTRON TRANSFER
ELECTRODES.


The present invention is concerned with the use of
carbon-boron compounds in electron-transfer electrodes
and assays which use the said electrodes. Although the
invention will be described with reference to a specific
assay for hydrogen peroxide, the invention extends inter
alia to assay systems and electrodes for use with a wide
range of enzyme substrates.


Metallocarborane complexes have been described for use
in catalysis, more specifically in homogenous
hydrogenation, isomerization and hydroformylation of
olefins and the hydrosilation of ketones. United States
patent 4363747 (December 14, 1982) describes a catalyst
of the type mentioned above.


Our European Patent Application No. 82305597 describes
and claims a sensor electrode composed of electrically
conductive material and comprising at least at an
external surface thereof the combination of an enzyme
and a mediator compound which transfers charge to the
electrode when the enzyme is catalytically active.

Such an electrode is referred to herein as "an electron-transfer electrode". The purpose of such an electrode is to detect the presence of, measure the amount of and/or monitor the level of one or more selected components capable of undertaking a reaction catalysed by the said enzyme.

Our European Patent Application discloses the use of the following classes of compounds as mediators:-

polyviologens
chloranil, fluoranil, bromanil and derivatives thereof
alkyl substituted phenazine derivatives
metallocenes.

Metalloboron compounds have been generally described in many papers, of which one particularly good reference for the purposes of the present specification is Geiger [Electrochemistry of Metalloborom cage compounds]. This paper compares and contrasts the electrochemistry of the carbon-boron compounds which contain transition metal atoms with the metallocenes, and in particular the ferrocenes. The paper describes cyclic voltammetry experiments with appropriate sweep rates for pen and ink recorders, but does not consider mediator chemistry.

3

We have now discovered the particular utility of a further class of mediators.

According to the present invention there is provided a mediator compound for use with an electron transfer electrode, the said compound containing carbon and boron.

Conveniently the mediator compound is a metallocarborane.

In general, the metallocarboranes have greater thermal stability and resistance to chemical attack that the respective metallocene analogues. More importantly they allow a much wider range of electrochemical potentials to be assessed.

The nomenclature of carboranes has recently undergone several changes. In order to alleviate confusion the nomenclature used herein is that found in the bulk of the literature, even though a purportedly systematic naming system now exists.

Preferably, the following metallocarboranes and complex ions are employed:-
1(a)  the bisdicarbollyl complexes of $d^3$, $d^5$, $d^6$, $d^7$, $d^8$ and $d^9$ transition metal ions,

These may include the species Fe(II), Fe(III), Co(II),

4

Co(III), Ni(III), Pd(III) and Cr(III), in the conventional configuraton of 'symetrical sandwich', the additional configuration of 'slipped sandwich' (as found with the $d^8$ and $d^9$ complexes of Cu(II), Cu(III), Ni(II), Pd(II), Au(II) and Au(III)), and the 'cisoid sandwich' configuration as found in the $d^6$ complexes of Ni(IV) and Pd(IV).

It is envisaged that the more obscure bisdicarbollyl complexes with ligand substituents such as m- and p-fluorophenyl, b-phenyl, C,C'-divinyl and C,C'-trimethylene could be employed within the scope of the present invention.

(b) mixed complexes in which a dicarbollyl ligand formally replaces a cyclopentadienyl ligand in a metallocene,

Treatment of a metal salt with dicarbollide ion and cyclopentadienyl ion produces, in most cases neutral M(III) complexes which result from air oxidation of the corresponding M(II) complex.

This type of complex has been prepared for Fe(II), Fe(III), Co(III), Ni(III) and Cr(III). At least one complex has been prepared which includes platinum (II), while at least two complexes have been prepared which

5

include palladium (II).

(c) bidentate and polydentate complexes containing the dicarbacanastide ligand, and

(d) dinuclear and polynuclear complexes containing one or more cyclopentadienyl ligands.

As with the previous mediator compounds these compounds are capable of transferring charge from an enzyme or enzyme complex to an electrode surface when the enzyme is catalytically active.

Enzyme/substrate pairs whose electrochemical behaviour in association with mediator compounds have been studied by the Applicants include the following:-

| Enzyme | Substrate |
| --- | --- |
| Flavo-proteins | |
| Pyruvate Oxidase | Pyruvates |
| L-Amino Acid Oxidase | L-Amino Acids |
| Aldehyde Oxidase | Aldehydes |
| Xanthine Oxidase | Xanthines |
| Glucose Oxidase | Glucose |
| Glycollate Oxidase | Glycollate |
| Sarcosine Oxidase | Sarcosine |
| Galactose Oxidase | Galactose |

0150602

6

| | |
|---|---|
| Diaphorase | NADH |
| Glutathione Reductase | NADPH |

PQQ Enzymes

| | |
|---|---|
| Glucose Dehydrogenase | Glucose |
| Methanol Dehydrogenase | Methanol and other Alkanols |
| Methylamine Dehydrogenase | Methylamine |

Cytochrome B-linked Enzymes

| | |
|---|---|
| Lactate Oxidase | Lactate |

Metalloflavoproteins

| | |
|---|---|
| Carbon monoxide Oxidoreductase | Carbon Monoxide |

It is believed that many of these enzyme-substrate pairs could be utilised in association with the mediator in the present invention, given some limitations on the assay conditions which would be obvious to the man skilled in the art. Of these pairs, it is clearly advantageous to utilise those enzyme/substrate pairs whose behaviour is established in most detail and which give good, preferably linear, response over the expected measurement range.

In order that the present invention is more clearly

7

understood it will be explained by way of example and with reference to the accompanying figures wherein;

Figure 1 shows compounds comprising suitable mediators within the ambit of the present invention,

Figure 2 shows an experimental cell for use in the method of the present invention,

Figure 3 shows a cyclic voltammogram of a carborane complex in the presence of horseradish peroxidase,

Figure 4 shows a cyclic voltammogram of a carborane complex in the presence of hydrogen peroxide,

Figure 5 shows a cyclic voltammogram of a carborane complex in phosphate buffer,

Figure 6 shows the current/time response curve on addition of hydrogen peroxide to a carborane/ horseradish peroxidase mixture, and,

Figure 7 shows the calibration graphs for the carborane/ horseradish peroxidase system

EXAMPLE 1; SUITABLE MEDIATORS

8

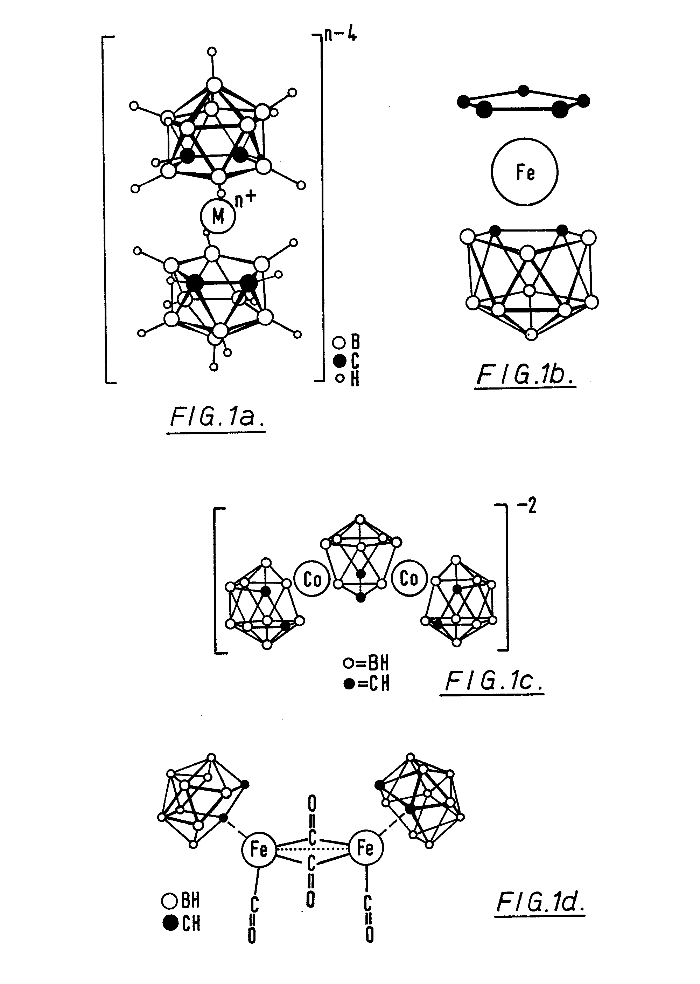

Figure 1 shows compounds comprising suitable mediators within the ambit of the present invention.

Specific examples of the above classes of compound are given as follows :

EXAMPLE 1(a)  $[(1,2-B_9C_2H_{11})_2Fe(II)]$  or  the chromium or nickel analogue, i.e. the compound;

An examples of the use of $[(1,2-B_9C_2H_{11})_2Fe(II)]$ is its use in coupling an electrode to dehydrogenases, especially those requiring co-factor regeneration such as those which employ NADH or NADPH. This compound has a low reduction potential of -0.174Volts, compared to ferrocene at +0.590Volts.

The nickel (IV) bisdicarbollyl complex can couple at +0.500 volts $E^O$ vs. N.H.E. (Hydrogen Electrode). The chromium analogue also has particular utility, as it is both electrochemically active and air stable.

EXAMPLE    1(b)     [$(1,2-B_9C_2H_{11})(C_5H_5)Fe(III)$],    and other compounds of the general type;

Examples    of    the    use    of    the    mixed    complex [$(1,2-B_9C_2H_{11})(C_5H_5)Fe(III)$]    are    as    a    mediator compound for use with flavoproteins, glucose oxidase, diaphorase and the quinoprotein glucose dehydrogenase.

This compound has be found to have a reduction potential of +0.130 Volts, which indicates that it will aid in the avoidance of interference from contaminants or additional substances which are present in physiological assay systems.

EXAMPLE 1(c)

$[(1,2-B_9C_2H_{11})Co(III)(B_8C_2H_{10})Co(III)(1,2-B_9$ $C_2H_{11})]^{2-}$; i.e. the compound;

O = BH
● = CH

EXAMPLE 1(d)

$[(1,2-B_9C_2H_{11})_2Fe_2(I)(CO)_4]^{2-}$, i.e. the compound;

◯ BH
● CH

Examples of the use of compounds containing more than one metal ion, of the same (as in case (c)) or different atomic numbers (as in case (d)), are in the fine tuning of mediators to specific enzyme reactions.

EXAMPLE 2; USE OF THE MIXED COMPLEX MEDIATOR COMPOUND $[(1,2-B_9C_2H_{11})(C_5H_5)Fe(III)]$, i.e. the compound of the general type;

○ B-H
● C-H

The pi-cyclopentadienyl-pi-dicarbollyl-iron complex, $CpFeC_2B_9H_{11}$ was investigated as a mediator between horseradish peroxidase and a gold electrode. In the system as described below, hydrogen peroxide acts as a substrate for horseradish peroxidase (type iv) (Sigma), the latter being converted to an oxidised intermediate.

Hydrogen peroxide (Aristar Grade, 30% w/v solution) was obtained from BDH (British Drug Houses) and a stock solution of strength approx. $10^{-3}$ Molar was prepared and assayed by an iodometric titration.

Before use the concentration of the stock horseradish peroxidase solution was estimated at $10^{-4}$ Molar, using a Pye Unicam SP8-200 UV/visible spectrophotometer and a molar extinction coefficient of $9.1*10^{4}M^{-1}cm^{-1}$ at 403nm as described by (D. Keilin and E. F. Hartree, Biochem. J 1951, 49, 88-104).

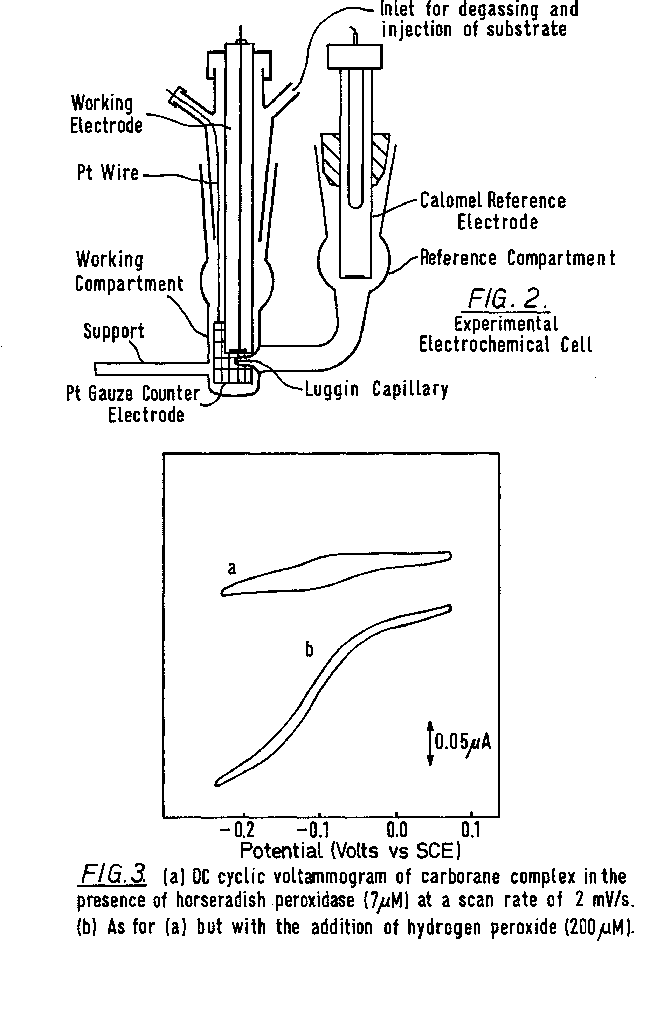

DC cyclic voltametry was performed in a two compartment glass cell (shown in figure 2) which contained a platinum gauze counter electrode and a saturated calomel reference electrode (SCE) as well as the gold working electrode. The working electrode was polished before use and between each experiment, with a water/alumina slurry having an approximate particle size of 0.3um and applied to the electrode surface with cotton wool. After polishing the elctrode was washed with distilled water.

For DC cyclic voltammetry a Bryans X-Y 26000 A3 chart recorder was used in combination with an Oxford Electrodes Potentiostat. Current-time curves were recorded with a Bryans Y-t 28000 recorder.

Argon-saturated quiescent solutions in deionised (Millipore) distilled water were used throughout. The range of potential scanned was $-220mV$ to $+60mV$.

The carborane was prepared according to the method of the literature given by Hawthorne et al (Hawthorne, Young, Andrews, Howe, Pilling, Pitts, Reintjes, Warren and Wegner, Journal of the American Chemical Society 1968, 90, 879-896)

Due to the lack of solubility of the complex in water it was necessary to disperse the solid in a small volume of Tween 20 (polyoxyethylene sorbitan monolaurate) (Sigma) by sonication prior to dilution with 0.05Molar phosphate buffer at pH 6.43.

Three experiments were performed, in which the initial concentrations of the reagents in the cell were;

Horseradish Peroxidase $7*10^{-6}$ Molar (in (a) and (c))
Hydrogen Peroxide $2*10^{-4}$ Molar
Tween 20 approx 1% by final volume.

In all cases the supporting electrolyte was 0.05 Molar phosphate buffered at pH 6.43. The concentration of the mediator was not determined

EXAMPLE 2(a)

A carborane, buffer and horseradish peroxidase mixture was placed in the electrochemical cell. Following deoxygenation with oxygen-free argon, a cyclic voltammogram was obtained at a slow scan rate (2 mV/s). 5 ul of hydrogen peroxide were then injected and scanning recommenced. A clear change in the voltammogram occurred, with the disappearence of peaks, and a large catalytic current flowed at reducing potentials. The cyclic voltammogram is shown in figure 3.

EXAMPLE 2(b)

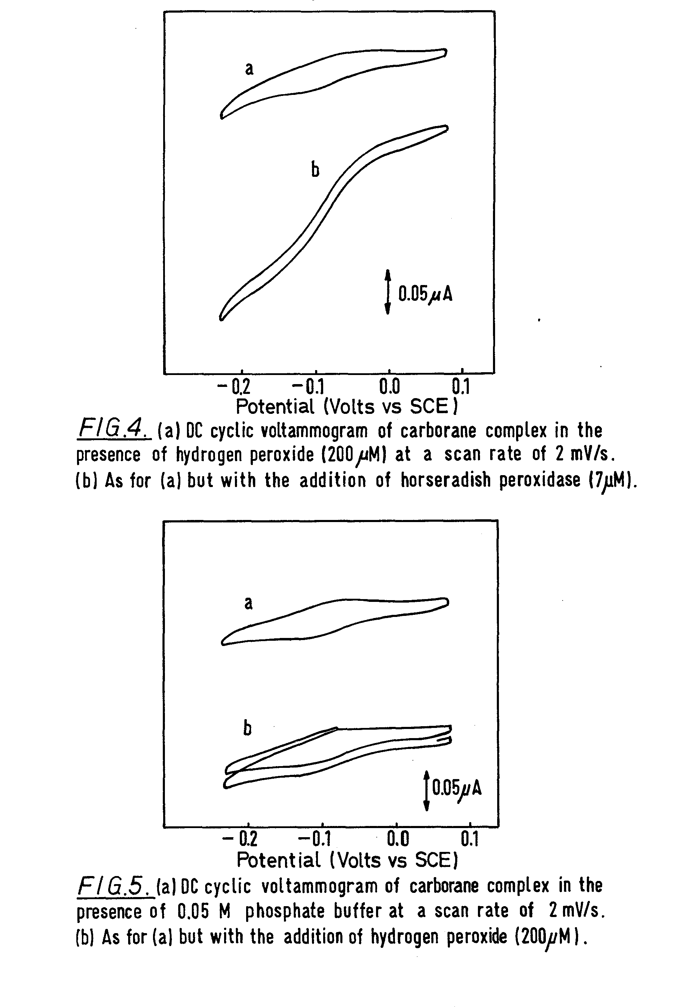

The same protocol was followed as in the previous experiment (2(a)) except that a carborane, buffer and hydrogen peroxide mixture was placed in the elctrochemical cell and the enzyme was injected. This had the same effect on the voltammogram as given above the results being shown in figure 4.

EXAMPLE 2(c)

A control experiment was carried out in which hydrogen peroxide was added to a carborane-buffer mixture (i.e.

no enzyme was present in the assay mixture). No change to the voltammogram was observed as can be seen from figure 4.

EXAMPLE 3

In order to illustrate the usefulness of the carborane horseradish peroxidase system in the quantitative determination of substrate (in this case hydrogen peroxide), the working electrode was poised at a constant potential of -138mV vs SCE and the response of the system was monitored on addition of hydrogen peroxide to the carborane-enzyme system.

Initial concentrations of components in the electrochemical cell were;

Horseradish Peroxidase $3*10^{-5}$ Molar, and,
Hydrogen Peroxide $1*10^{-7}$ to $1*10^{-6}$ Molar,

other experimental conditions were as previously stated.

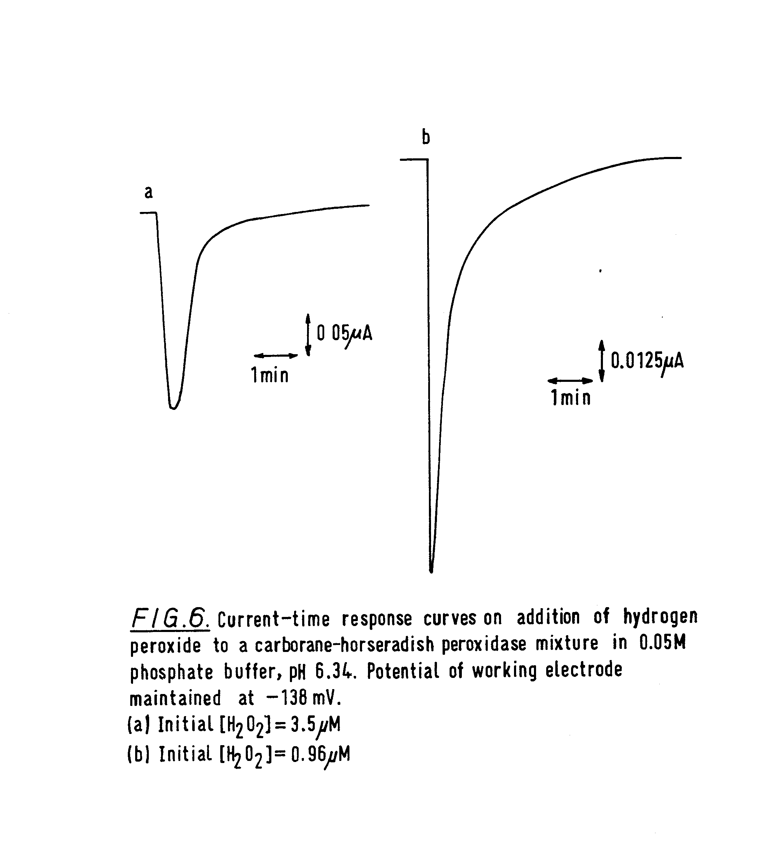

Current against time curves of the type as shown in figure 6 were obtained. The data was processed in two ways.

Firstly, the peak height, which is indicative of the catalytic current, was calculated and plotted against the concentration of hydrogen peroxide added by injection.

Secondly, the area under the current/time curve, indicative of the total current passed, was calculated and plotted against the hydrogen peroxide concentration.

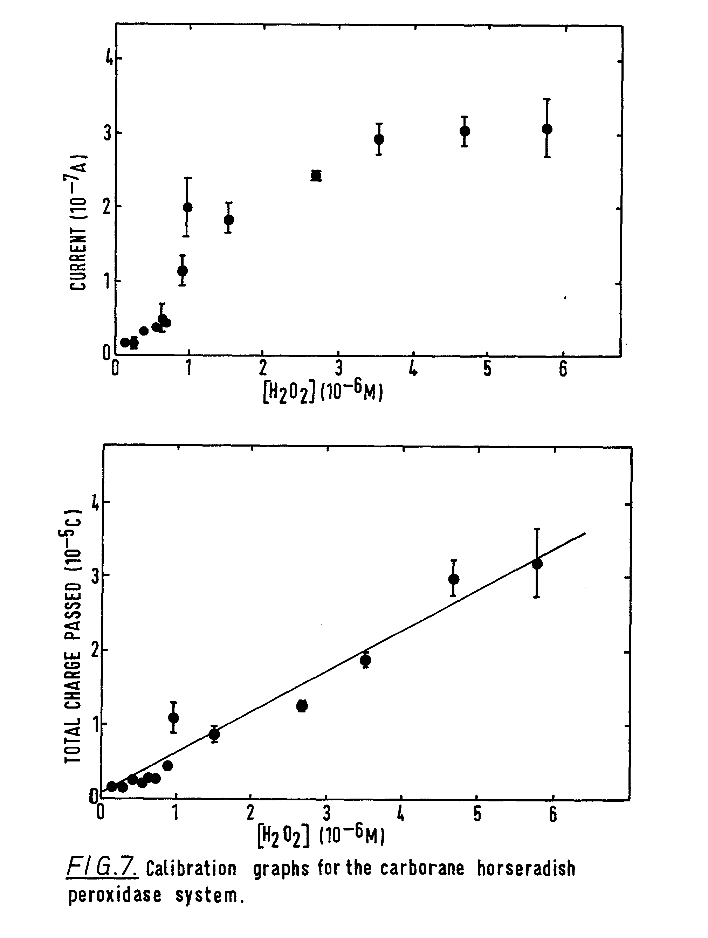

The two calibration curves obtained by the above process are reproduced in figure 7.

The experiment demonstrates the use of the compound $CpFeC_2B_9H_{11}$ as a mediator (where $Cp=C_5H_5$) in a quantitative assay for hydrogen peroxide.

Although the present invention has been described in terms of specific mediator compounds, and by a specific example, the invention also extends to:

(a) electrodes, as defined above, with the various mediator and enzyme combinations,

(b) cells defining a liquid chamber and incorporating such an electrode and a reference electrode,

(c) equipment including such cells,

(d)  assay  methods  including  the  use  of  carborane
containing mediator compounds, and


(e)  a  chemically-linked  mediator-enzyme  combination,
mediator-enzyme-antibody                           combination,
mediator-enzyme-antigen  combination,  or  mediator-DNA
combination  where  the  mediator  is  a  carborane  or  a
carborane derivative.

CLAIMS

1) The use of a mediator compound in combination with an electron transfer electrode,wherein the said mediator compound containing carbon and boron.

2) The mediator of claim 1, wherein the compound is a metallocarborane.

3) The mediator of claim 1 or 2, wherein the mediator is selected from the group comprising the bisdicarbollyl complexes of $d^3$, $d^5$, $d^6$, $d^7$, $d^8$ and $d^9$ transition metal ions,

4) The mediator of claim 2, wherein a dicarbollyl ligand formally replaces a cyclopentadienyl ligand in a metallocene.

5) The mediator of claim 2, wherein the mediator is a bidentate or polydentate complex containing the dicarbacanastide ligand.

6) The mediator of claim 2, wherein the mediator is a dinuclear or polynuclear complex containing one or more cyclopentadienyl ligands.

7) The mediator of claim 3 comprising the compound $[(1,2-B_9C_2H_{11})_sFe(II)]$ or the chromium or nickel analogue.

8) The mediator of claim 4 comprising the compound $[(1,2-B_9C_2H_{11})(C_5H_5)Fe(III)]$.

9) The mediator of claim 5 comprising the compound $[(1,2-B_9C_2H_{11})Co(III)(B_8C_2H_{10})Co(III)(1,2-B_9C_2H_{11})]^{2-}$

10) The mediator of claim 6 comprising the compound $[(1,2-B_9C_2H_{11})_2Fe_2(I)(CO)_4]^{2-}$

11) The mediator of claim 6 comprising the compound $CpFeC_2B_9H_{11}$.

12) A mediator compound as claimed in any of the preceeding claims in association with an enzyme, wherein charge may be transferred between the enzyme and the mediator when the enzyme is catalytically active.

13) The mediator compound of claim 12 in association with an electrically conducting surface.

14) The mediator of claim 11, in an assay reagent including horseradish peroxidase.

20

15) An assay reagent comprising a mediator as claimed in any one of claims 1-11, and an enzyme.

16) An electrode for use in an assay, comprising a mediator as claimed in any one of claims 1-11 and an electrically conducting body, the arrangement being such that charge is capable of being transferred from the mediator to the electricaly conducting body.

FIG.1a.

FIG.1b.

FIG.1c.

FIG.1d.

0150602

FIG. 2.
Experimental
Electrochemical Cell

Inlet for degassing and
injection of substrate

Working
Electrode

Pt Wire

Calomel Reference
Electrode

Reference Compartment

Working
Compartment

Support

Pt Gauze Counter
Electrode

Luggin Capillary

Potential (Volts vs SCE)

FIG.3. (a) DC cyclic voltammogram of carborane complex in the
presence of horseradish peroxidase (7μM) at a scan rate of 2 mV/s.
(b) As for (a) but with the addition of hydrogen peroxide (200μM).

**FIG.4.** (a) DC cyclic voltammogram of carborane complex in the presence of hydrogen peroxide (200 μM) at a scan rate of 2 mV/s. (b) As for (a) but with the addition of horseradish peroxidase (7μM).

**FIG.5.** (a) DC cyclic voltammogram of carborane complex in the presence of 0.05 M phosphate buffer at a scan rate of 2 mV/s. (b) As for (a) but with the addition of hydrogen peroxide (200μM).

FIG.6. Current-time response curves on addition of hydrogen peroxide to a carborane-horseradish peroxidase mixture in 0.05M phosphate buffer, pH 6.34. Potential of working electrode maintained at −138 mV.
(a) Initial [$H_2O_2$] = 3.5 $\mu$M
(b) Initial [$H_2O_2$] = 0.96 $\mu$M

FIG.7. Calibration graphs for the carborane horseradish peroxidase system.